# EUROPEAN PATENT APPLICATION

(11) **EP 4 424 175 A1**
(43) Date of publication of application: **04.09.2024**
(21) Application number: 23159953.1
(22) Date of filing: 03.03.2023
(51) Int. Cl.: A23L 33/105, A23L 33/12, A23L 33/15, A61K 36/02, A23D 9/007, A23D 9/04, A23L 33/155

(54) **DIETARY SUPPLEMENT BASED ON OMEGA-3 ACIDS OF VEGETABLE ORIGIN AND A FORM OF VITAMIN D**

(71) Applicant: Enervit S.p.A., 20149 Milano (IT)
(72) Inventor: PETRONI, Paolo, 20833 GIUSSANO (IT); MUSAZZI, Diego, 20025 LEGNANO (IT)
(74) Representative: Cattaneo, Elisabetta

(57) **Abstract**

The present invention concerns a composition based on at least one omega-3 polyunsaturated fatty acid (n-3 PUFA) preferably of non-animal origin in combination with a hydroxylated form of vitamin D. The combination of the components of the composition results in a synergistic anti-inflammatory action on the human organism that makes it suitable for use in the prevention or treatment of an inflammatory disease, in particular of the chronic type.

## Description

### FIELD OF THE INVENTION

The invention concerns a composition and a supplement based on a combination of omega-3 acids of vegetable origin and a hydroxylated form of vitamin D. The supplement is suitable in the treatment and in the prevention of an inflammatory state of the human organism or of an inflammatory disease, in particular of a chronic type. The present invention originates in the field of dietary supplements and nutraceutical/functional products, and in particular it concerns a composition based on selected active ingredients of non-animal origin whose synergistic action allows to reduce the inflammatory state of the human organism or to treat an inflammatory disease, in particular chronic.

### BACKGROUND ART

Inflammation is a defence mechanism of the body that is activated in the presence of harmful events of an irritative, allergic or infectious type. Inflammation is an evolutionarily conserved process, characterized by the activation of cells belonging mainly to the immune system, which protect the body from bacteria, viruses and toxins by eliminating pathogens and promoting tissue repair (Netea MG et al., Nat. Immunol. 18, 826-831 (2017). The inflammatory response is activated by the body whenever it perceives a threat, and, normally, when the factor perceived as harmful has been eradicated, it starts the process of "turning off' the inflammation, defined as resolution. Usually, the inflammatory response is well regulated and tends not to cause excessive damage to the host. In this case, inflammation is defined as self-limiting and is rapidly resolved thanks to the activation of negative feedback mechanisms, through secretion of anti-inflammatory cytokines or resolving lipid mediators, through inhibition of the pro-inflammatory cascade or through the activation of regulatory cells (Kotas ME & Medzhitov R. Cell 160, 816-827 (2015). Calder PC et al. Biochim Biophys Acta 1851, 469-484 (2015).)

The body's initial response to a harmful stimulus occurs through acute inflammation and involves an increased movement of plasma and leukocytes from the blood towards the damaged tissue. Then a cascade of biochemical events that matures the inflammatory response follows: these events are a prerogative of the local vascular system, of the immune system and of several cells of the damaged tissue. Under normal conditions, the inflammation tends to resolve quickly, in a couple of hours or in a couple of days; however, in case it is prolonged excessively, the inflammation is defined as chronic and can last even for years. Regardless of the type of event that caused the inflammation to arise, the response initially comprises an increase in blood flow towards the inflamed site, followed by an increase in the permeability of the vascular wall due to a greater opening of the junctions between endothelial cells, which allows the passage of soluble mediators at the site of inflammation. Then the leukocytes migrate from the bloodstream towards the surrounding tissue, a process that is promoted by the release of chemo-attractors from the site of inflammation and by the increase in the production of adhesion molecules at the endothelium level. Finally, the leukocytes release different types of chemical mediators at the site of inflammation depending on the cell type involved, on the nature of the inflammatory stimulus, on the anatomical site, and on the stage of the inflammatory process. Among the chemical mediators involved there may be those of lipid derivation (prostaglandins, leukotrienes, endocannabinoids, platelet activation factor), peptide mediators (cytokines, chemokines), reactive oxygen species, amino acid derivatives (histamine) and enzymes (matrix proteases) (Calder PC. Nutrients 2(3), 355-374 (2010).

Acute inflammation arises primarily from a bacterial or viral infection through an interaction between cellular receptors expressed on cells of the innate immune system and conserved structures present on the membranes of pathogens, called pathogenassociated molecular patterns (PAMP). The acute response can also be activated by "sterile" agents, defined as molecular damage-associated patterns (DAMP), which are released in response to harmful stimuli of a physical, chemical or metabolic nature during stress or cell damages (Furman D et al. Nat. Med. 25, 1822-1832 (2019).

A chronic inflammatory state is instead activated by DAMP in the absence of an acute infectious or harmful event. Among the triggers of a state of systemic inflammation, there may be the presence of biological, environmental, social and psychological factors, which often inhibit the complete resolution of the acute inflammatory state, thus promoting a state of chronic inflammation of a systemic nature. The transition from an acute to a chronic inflammatory response can be followed by severe functional and structural alterations in all tissues and organs, which, by altering the normal cell physiology, can increase the risk of chronic diseases in both adult and young populations. Chronic inflammation can also alter normal immune function, increasing susceptibility to infections, tumours, and decreasing response to vaccinations (Shen-Orr SS et al. Cell Syst. 3, 374-384 (2016).

Basically, with increasing age, the markers of the chronic systemic inflammation also increase: higher levels of cytokines, chemokines and acute phase proteins are found, as well as greater expression of genes involved in inflammation. The establishment of the chronic systemic inflammation can cause over time several collateral damages to tissues and organs, leading to the development of severe pathological conditions such as metabolic syndrome, type 2 diabetes, cardiovascular diseases, different types of cancer, autoimmune and neurodegenerative diseases, consequently reducing the quality and life expectancy of people who are affected by them (Franceschi C et al. Trends Endocrinol. Metab. 28, 199-212 (2017).

Therefore, it is appropriate to evaluate the intake of long-term supplements, which, together with some targeted changes in lifestyle such as diet, physical activity, stress, can improve, both preventively and therapeutically, the inflammatory status of an individual by limiting the negative health outcomes (Furman D et al. Nat. Med. 25, 1822-1832 (2019).

The need is therefore currently felt to provide products suitable for reducing the development of the inflammation, in particular chronic inflammation, whose dispensation to the public is not subject to medical prescription and whose administration is free of side effects also in view of the fact that the lifestyles of modern society make inflammation of the organism in increasingly young populations more and more frequent.

### SUMMARY OF THE INVENTION

The Applicant of the present invention has observed that a specific composition comprising at least one omega-3 polyunsaturated fatty acid (n-3 PUFA) in combination with calcifediol, a hydroxylated form of vitamin D, is endowed with beneficial properties in the absence of side effects, in the treatment and in the prevention of an inflammatory state of the human organism or of an inflammatory disease.

The invention therefore concerns a composition for oral administration comprising at least one omega-3 polyunsaturated fatty acid (n-3 PUFA) in combination with calcifediol, and a physiologically acceptable carrier.

The dependent claims appended herein describe embodiments of the invention.

### DETAILED DESCRIPTION OF THE INVENTION

The present invention originates from having surprisingly identified a composition comprising at least one omega-3 polyunsaturated fatty acid (n-3 PUFA) in combination with calcifediol endowed with beneficial properties in the absence of side effects, in the treatment and in the prevention of an inflammatory state of the human organism or of an inflammatory disease.

The invention, therefore, in a first aspect, concerns a composition for oral administration comprising at least one omega-3 polyunsaturated fatty acid (n-3 PUFA) in combination with calcifediol, and a physiologically acceptable carrier.

In a preferred embodiment, said at least one omega-3 polyunsaturated fatty acid is selected from the group consisting of α-linolenic acid (ALA), stearidonic acid (SDA), eicosatrienoic acid (ETE), eicosatetraenoic acid (ETA), eicosapentaenoic acid (EPA), docosapentaenoic acid (DPA), tetracosapentaenoic acid, tetracosahexaenoic acid and docosahexaenoic acid (DHA) and mixtures thereof, preferably EPA, DHA and mixture thereof.

In an even more preferred embodiment, the at least one omega-3 polyunsaturated fatty acid is DHA. In a further preferred embodiment, the at least one omega-3 polyunsaturated fatty acid has a DHA/EPA ratio which may range from 5000:1 to 1 :5000, preferably from 50:1 to 1:50.

Omega-3 polyunsaturated fatty acids are, together with omega-6, part of the category of essential fatty acids. They are best known for their presence in cell membranes and for maintaining the integrity thereof, and have very different roles in the inflammatory response. Omega-6, substances similar to hormones, start the inflammatory process to protect the body from different types of offences such as, for example, microbial invasions. Among omega-6s, arachidonic acid is the major precursor to a wide variety of pro-inflammatory eicosanoids. Its dosages in the blood should be between 7% and 9% of the total fatty acids, but due to a wrong diet these levels are often higher, contributing to a higher inflammatory state.

Eicosanoids are key lipid mediators of inflammation, include oxidized derivatives of 20-carbon polyunsaturated fatty acids, and include prostaglandins, thromboxanes and leukotrienes. The main precursor of the eicosanoids is the arachidonic acid, which is released from the membrane phospholipids of the inflammatory cells through the action of phospholipase A2, an enzyme that is activated by the inflammatory stimulus. Arachidonic acid acts as a substrate for the cyclo-oxygenase (COX), lipooxygenase (LOX) and cytochrome P450 enzymes, which produce pro-inflammatory mediators such as PGE2 and LTB4. Eicosanoids act through specific receptors, such as the receptors coupled to protein G. Among the eicosanoids, mention can be made of the endocannabinoids, produced starting from phospholipids whose bonds have been cut by phospholipases other than A2. Endocannabinoids exert anti-inflammatory action, and can be produced starting from the omega-3 fatty acids EPA and DHA.

It has been observed that, following the use of omega-3-based supplements, there is a reduced production of prostaglandins and leukotrienes from arachidonic acid. Omega-3s make up the fundamental components of the group of hormones called resolvins, which help resolve the inflammatory process. The intake of omega-3s containing EPA and DHA tends to shift the profile of the membrane fatty acids towards a lower content of arachidonic acid, and consequently a lower production of inflammatory eicosanoids occurs. Typically, the omega-3 polyunsaturated fatty acids exert an anti-inflammatory effect by regulating the production of pro-inflammatory cytokines (TNF, IL-1b, IL-6). In particular, n-3 PUFAs, preferably EPA and DHA, are capable of inhibiting the production of TNF, IL-1b and IL-6 by macrophages. Some studies on animal models have shown an effect of the omega-3s in increasing the levels of the anti-inflammatory cytokine IL-10. Similar results were also found in human subjects, where it is assumed that the mechanism involved may be a reduction in gene expression of the pro-inflammatory cytokines, by action on the pathways NFkB and PPAR-γ.

This mechanism of regulating the inflammation leads to reduced levels of circulating pro-inflammatory cytokines. In addition, potent anti-inflammatory metabolites such as resolvins, maresins, and protectins, collectively called specialized pro-resolution mediators, are derived from omega-3 metabolism. The biological effects of resolvins, protectins and maresins have been extensively examined in cell cultures and animal models of inflammation, demonstrating an anti-inflammatory and inflammation-resolving effect. Resolvins are able to inhibit the infiltration and the transendothelial migration of neutrophils, cells belonging to the innate immune response. In addition, the resolvins inhibit the production of pro-inflammatory mediators, and have a potent anti-inflammatory and immunoregulatory effect. This leads to less activation of the inflammatory cascade, allowing the inflammation to be controlled. Pro-resolution molecules work by activating specific mechanisms that promote homoeostasis. Specifically, these molecules stimulate resolution through multifactorial mechanisms at the tissue interface. Specific resolvins and protectins provide a powerful stimulus that selectively blocks the infiltration of neutrophils and eosinophils. In addition, they stimulate the recruitment of monocytes without pro-inflammatory mediators, activate macrophage phagocytosis, increase phagocyte removal, and stimulate the expression of antimicrobial defence mechanisms. Several studies have also pointed out an effect on the resolution of the inflammation through MAPK and NF-kB cell signalling pathways, thus performing an anti-inflammatory effect based on gene transcription regulation. It has also been observed that omega-3 supplementation leads to a reduction in neutrophil and monocyte chemotaxis towards several chemo-attractors such as LTB4, bacterial peptides and human serum. Since chemotaxis is the process by which leukocytes move to the site of inflammation in response to the release of molecules called chemo-attractors, the result of this omega-3 mediated regulation mechanism is a lower presence of pro-inflammatory cells on the site of inflammation, and consequently a lower activation of the inflammatory cascade.

Studies on cell cultures and animal models have shown that omega-3s can reduce the expression of certain adhesion factors on the surface of monocytes, macrophages, lymphocytes, and endothelial cells, effectively reducing the adhesive interactions between monocytes and endothelial cells. The adhesion factors are proteins that are expressed on the surface of the endothelial cells and leukocytes. These molecules promote the interaction between different cell types: it is thanks to these interactions that the leukocytes in the blood can interact with the walls of the vessels and cross the endothelial junctions to reach the site of inflammation. In human subjects, EPA + DHA supplementation has led to lower levels of ICAM-1 adhesion factor expression on the surface of IFN-γ-stimulated monocytes.

This mechanism has a combined and additive effect with the reduction of chemotaxis: fewer leukocytes reaching the site of inflammation means less secretion of pro-inflammatory mediators and consequently greater control over the inflammatory response.

Taken together, these results demonstrate how omega-3s can cause a shift from a strongly pro-inflammatory environment to a state of reduced inflammation, with lower cellular reactivity and higher resolution of inflammation.

According to some aspects of the present invention, the at least one omega-3 polyunsaturated fatty acid is not of animal origin and is preferably contained in an oil of non-animal origin, advantageously containing DHA and EPA.

In the present invention when the term "non-animal oil" is used it is meant to comprise both vegetable and synthetic oils.

In a preferred embodiment, the oil of non-animal origin is obtained by extraction from a microalga, preferably *Schizochytrium sp.* Therefore, the oil of non-animal origin is preferably a vegetable oil.

In a preferred embodiment, the composition of the invention also comprises arachidonic acid. It is preferably contained in the non-animal oil. The arachidonic acid content in the non-animal oil is preferably less than 20 mg, more preferably it is comprised from 1 mg to 13 mg per gram of oil.

The use of an oil of non-animal origin in the composition described herein has numerous advantages. Many studies in the literature have confirmed the presence of extremely low levels of arachidonic acid in the DHA-containing oils of vegetable origin. For example, the arachidonic acid content in the analysed oils from microalgae *Schizochytrium sp.* containing DHA is between 1 mg and 13 mg per gram of oil, while in commonly commercially available fish oil the arachidonic acid levels exceed 20-25 mg per gram of oil (Yokochi T. Appl Microbiol Biotechnol 49, 72-76 (1998), Zhu L. Proc Biochem 42, 210-214 (2007), Zou X. Appl Biochem Biotech 191, 1294-1314 (2020), EFSA Journal 2020;18(10):6242, EFSA Journal 2021;19(1):6345, Dossier Novel Food Martek (2010)).

The lower arachidonic acid content of the DHA-containing oil of vegetable origin used in the context of the invention, compared to the animal source oil commonly present on the market therefore guarantees an even greater anti-inflammatory potential to the composition of the invention, thanks precisely to the lower arachidonic acid content known for its pro-inflammatory effects.

The use of omega-3s of non-animal origin also helps to solve food safety problems typical of omega-3s of animal origin. In particular, the fish allergen is completely absent in non-animal oil, and this allows for the intake of omega-3s in total safety even in individuals allergic to fish and derivatives. The vegetable origin also makes it possible to limit environmental problems related to the procurement of raw material from "nonrenewable" animal sources. By limiting the human impact on the marine ecosystem, it also allows to preserve the natural ecosystem of this environment, and opens the doors to a wider production of omega-3s to allow a sustainable integration of as many people as possible. Although the omega-3 supplements obtained from high-quality fish have very low levels of marine contaminants, the microalgae grown in closed tanks or industrial fermenters are completely free of heavy metals and other contaminants typical of the marine environment. This also solves the potential problem of bioaccumulation of certain contaminants that are not metabolised and tend to accumulate over time within the body.

Advantageously, the long-chain omega-3-based oil of vegetable origin used in formulating the composition of the invention differs from a long-chain omega-3-based oil of animal origin in the different structural stereochemistry of the triglyceride molecules that make up the oil.

In addition, the vegetable oil contains triglycerides with certain fatty acids in certain preferential positions, clearly different from what can be observed in animal oil. In particular, in the vegetable oil obtained from the microalga *Schizochytrium sp.* it can be observed that the fatty acid DHA is distributed equally in all 3 positions of the triglyceride, while the central position of the triglyceride (sn-2) is preferably occupied by myristic acid (Zhang T. J Am Oil Chem Soc 93, 1337-1346 (2016). In fish oil, on the other hand, regardless of the species chosen, it has been found that the fatty acid DHA is distributed preferentially in the central position of the triglyceride (sn-2), while the fatty acid EPA is distributed equally in all 3 positions of the triglyceride (Zhang H. Biomed Res Int 3529682, 1-10 (2018), Suarez ER. J Am Oil Chem Soc 87, 1425-1433 (2010).

In addition, the oils from different species show a different triglyceride spectrum. In particular, the triglyceride profile in the vegetable-origin oil is completely different from the triglyceride profile in the animal-origin oil.

For these reasons the use of an oil of a non-animal, preferably vegetable, nature in the composition of the invention is appreciable.

Another component of the composition is calcifediol, a particular form of biologically active vitamin D3. Vitamin D3 (cholecalciferol) is the natural form of vitamin D and is produced in the skin starting from 7-dehydrocholesterol. Following solar irradiation, 7-dehydrocholesterol is transformed into pre-vitamin D3, which undergoes a structural rearrangement to form vitamin D. This synthetic pathway represents the most important source of vitamin D for humans, and depends on the intensity of ultraviolet radiation. Vitamin D can also be taken through the diet, but it is usually present in a few types of foods including fish.

Vitamin D3 in itself is not biologically active: once synthesized it is transported in the liver through the bloodstream, and in the liver it is hydroxylated to produce 25-hydroxy-vitamin D3, the main circulating form of vitamin D, commonly known as calcifediol. At the renal level, the actual activation of vitamin D takes place with the conversion into 1,25-dihydroxy-vitamin D, known in particular for its role in the correct metabolism of calcium.

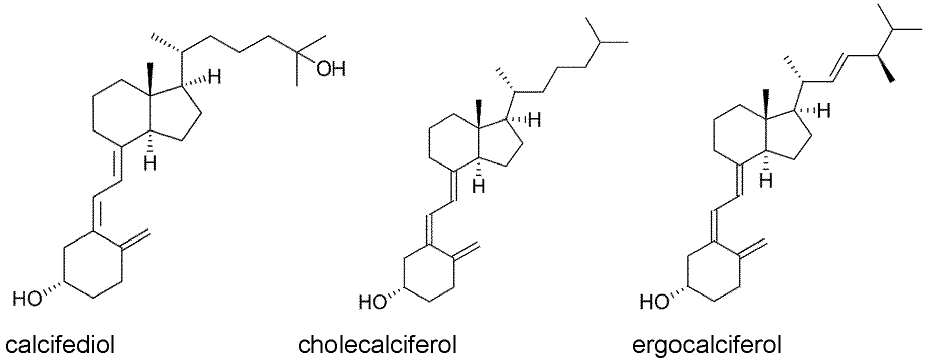

The active form of vitamin D (1,25-dihydroxy-vitamin D) has a potent anti-inflammatory action due to a switch from the phenotype of T *helper* immune cells, Th1/Th17, of pro-inflammatory nature, to the mainly anti-inflammatory Th2/Treg phenotype. The T *helper* Th1 and Th17 cells, which intervene in the adaptive immune response, have a strongly pro-inflammatory action profile, aimed at combating the pathogens through the secretion of pro-inflammatory cytokines such as IFN-γ, TGF-b, IL-6. In contrast, the response of Th2 and Treg cells is mainly anti-inflammatory, thanks to the secretion of anti-inflammatory cytokines such as IL-4, IL-10 and IL-2, and to the ability, typical of Treg cells, to maintain an immune homoeostasis avoiding phenomena of self-reactivity (Krajewska M. Front. Endocrinol. 13:920340 (2022).

Vitamin D is able to impair the development of Th17 cells starting from the undifferentiated precursor CD4, while increasing at the same time the development of regulatory cells capable of secreting IL-10. Inhibition occurs through the activation of the transcription factor VDR, which turns off the gene expression of RORγt thus inhibiting the development of Th17 cells (Palmer MT. J. Biol. Chem. 286(2), 997-1004 (2011). This change in the profile of the T cell population leads to a decreased secretion of pro-inflammatory mediators, such as IFN-γ, TNF-a, IL-1b, IL-6, IL-8, IL-12, IL-17 and an increase in the production of anti-inflammatory cytokines such as IL-4 and IL-10.

In addition, it has been observed that vitamin D protects stable levels of the anti-inflammatory cytokine IL-10.

Several in vitro studies and studies on animal model have also pointed out a potent anti-inflammatory effect of vitamin D in its activated form. The overall effect is, as already reported above, the transition from a pro-inflammatory state to a state with lower inflammatory potential (Guillot X. Joint Bone Spine 77, 552-557; 2010).

For these reasons, vitamin D supplementation has long been known and used in the world of supplements and functional foods. One of the problems encountered is that it is not always possible to correct a deficiency in a short time, especially in certain environmental conditions, for example because of a limited sun exposure. In terms of diet, few foods are a suitable source of natural vitamin D, especially in the context of some dietary choices such as typical Western or vegan diets.

The vitamin D supplements available on the market provide cholecalciferol, which however can take even months to reach the levels necessary to correct the most significant deficiencies.

Most vitamin D supplements of vegetable origin available on the market instead provide ergocalciferol, whose effectiveness has been described in the literature as further and significantly inferior to that of cholecalciferol. These factors lead to two important problems. On the one hand, in the absence of supplementation, vitamin D intake is often insufficient to meet the body's needs under certain environmental conditions. On the other hand, even in the presence of supplementation, it has been observed that normal cholecalciferol-based vitamin D supplements can even take several months to reach optimal circulating levels of vitamin D.

Hence the need for a more effective form of vitamin D in reaching optimal levels. This precursor, defined as calcifediol, originates during the *in vivo* activation of vitamin D in the liver. Subsequently, calcifediol is further processed in the kidneys giving rise to the biologically active form of vitamin D, 1,25-dihydroxy-vitamin D. The calcifediol used in the invention is also of non-animal origin.

Advantageously calcifediol is three times more potent than cholecalciferol and is also able to reach the bloodstream three times faster than cholecalciferol. This would allow not only to obtain optimal levels of vitamin D with lower dosages, but also to reach these levels in a much shorter time.

The composition according to the present invention combines omega-3 polyunsaturated fatty acids (n-3 PUFA) with calcifediol providing a synergistic anti-inflammatory effect making it suitable for use in the treatment and in the prevention of an inflammatory state of the human organism or of an inflammatory disease, preferably systemic chronic inflammation.

In a preferred embodiment, the composition of the present invention comprises an oil of non-animal origin having an overall EPA and DHA content in the range from 250 mg to 3000 mg, and calcifediol in the range from 0.83 to 16.7 µg.

The composition of the invention may take a wide variety of preparation forms. For example, the composition of the invention may be in solid form. In the case of preparations in solid form such as, for example, soft-gel or hard-gel capsule, bar, gummy candy, powder to be dissolved in water or ready-to-drink liquid emulsion.

Preparations in solid form may comprise one or more additives of a technological nature such as, for example, strength agents (e.g. glycerol), thickeners (e.g. carrageenan), acidity regulator (e.g. sodium hydrogen carbonate), tocopherol, structural agents (e.g. hydroxypropyl methyl cellulose, pectin, starches), gellan, lecithins, silicon dioxide, glycerol monostearate, polysorbate 80, flavourings, acidifiers (e.g. citric acid), acidity regulators (e.g. sodium citrate), fruit juice from concentrate, high oleic sunflower oil, rosemary extract, ascorbyl palmitate, dyes and/or fruit and vegetable concentrates, coating agents (e.g. carnauba wax), humectants (e.g. sorbitol), sweeteners (e.g. sucralose, stevia, erythritol).

According to some aspects of the invention the composition may be formulated as a dietary supplement.

The supplement therefore in addition to the composition of the invention also comprises suitable excipients for the final formulation.

The supplement according to the present invention may take a wide variety of forms depending on the form of preparation suitable for oral administration e.g. tablets, capsules, powders. One or more of the usual carriers used in the nutritional field and one or more excipients may be employed in the preparation of the compositions for the oral administration form.

In a preferred embodiment the composition or the supplement according to the present invention is for use in the treatment and in the prevention of an inflammatory state of the human organism or of an inflammatory disease, preferably the inflammatory state or the inflammatory disease is chronic.

### EXPERIMENTAL PART

### Example 1: composition of the invention and its preparation

For the preparation of the composition of the invention and therefore of the final product, such as for example a supplement, the following were employed:
- Oil of vegetable origin extracted from microalgae belonging to the species *Schizochytrium sp.* based on long-chain omega-3 containing EPA and DHA in a 1:2 ratio, in amounts of 525 mg per dose, such as to provide 250 mg of EPA + DHA per dose, plus the following excipients of a technological nature: high oleic sunflower oil, rosemary extract, tocopherols, ascorbyl palmitate.
- Calcifediol of non-animal origin, whose content per dose is equal to 4.15 µg, equivalent to 500 IU of "standard" vitamin D.
- Capsule, consisting of structural elements and additives of vegetable or non-animal origin in varying quantities: maize starch, strength agent: glycerol, thickener: carrageenan, acidity regulator: sodium hydrogen carbonate, tocopherol.

The production process for this soft-gel format is articulated as follows:
- Extraction of omega-3-rich algal oil from microalgae, in this case from algae of the species *Schizochytrium sp.*
- Purification of the algal oil: the extracted oil is purified to remove impurities and contaminants, such as heavy metals and toxins.
- Omega-3 concentration: the purified algal oil is concentrated to increase the content of omega-3 fatty acids.
- Mixing with other ingredients: other ingredients, such as antioxidants and stabilizers, to improve the shelf-life of the product, and calcifediol of non-animal origin are added to the purified and concentrated oil.
- Encapsulation: the mixture containing the omega-3-rich algal oil and calcifediol is placed inside soft-gel capsules of non-animal origin respecting the amounts indicated in example 1.
- Drying and packaging: the capsules are dried to remove any residual moisture and are then packaged for final distribution.

## Claims

1. Composition for oral administration comprising at least one omega-3 polyunsaturated fatty acid (n-3 PUFA) in combination with calcifediol, and a physiologically acceptable carrier.

2. Composition according to claim 1 wherein said at least one omega-3 polyunsaturated fatty acid is selected from the group consisting of α-linolenic acid (ALA), stearidonic acid (SDA), eicosatrienoic acid (ETE), eicosatetraenoic acid (ETA), eicosapentaenoic acid (EPA), docosapentaenoic acid (DPA), tetracosapentaenoic acid, tetracosahexaenoic acid and docosahexaenoic acid (DHA) and mixtures thereof, preferably EPA, DHA and mixture thereof.

3. Composition of claim 1 or 2 wherein the at least one omega-3 polyunsaturated fatty acid is DHA.

4. Composition according to anyone of claims 1-3 wherein the at least one omega-3 polyunsaturated fatty acid is not of animal origin.

5. Composition according to anyone of claims 1-4 wherein the at least one omega-3 polyunsaturated fatty acid is contained in an oil of non-animal origin, preferably containing DHA.

6. Composition according to claim 5 wherein the oil of non-animal origin is obtained from a microalga, preferably *Schizochytrium sp.*

7. Composition according to anyone of claims 1 to 6 wherein said composition also comprises arachidonic acid, preferably contained in the non-animal oil in an amount of less than 20 mg, more preferably in an amount in the range from 1 mg to 13 mg per gram of non-animal oil.

8. Dietary supplement comprising the composition according to anyone of claims 1-7 and excipients.

9. Composition according to anyone of claims 1-7 or dietary supplement according to claim 8, for use in the treatment or in the prevention of an inflammatory state of the human organism or of an inflammatory disease.

10. Composition for use according to claim 9 wherein the inflammatory state or inflammatory disease is chronic.
